# EUROPÄISCHE PATENTSCHRIFT

(11) **EP 2 163 617 B1**
(45) Veröffentlichungstag und Bekanntmachung des Hinweises auf die Patenterteilung: **21.03.2012**
(21) Anmeldenummer: 09167772.4
(22) Anmeldetag: 13.08.2009
(51) Int. Cl.: C12N 11/00, C08G 77/04

(54) **Enzympräparate**
Enzyme preparations
Préparations d'enzymes

(30) Priorität: 02.09.2008 DE 102008041754
(43) Veröffentlichungstag der Anmeldung: 17.03.2010
(73) Patentinhaber: Evonik Goldschmidt GmbH, 45127 Essen (DE)
(72) Erfinder: Thum, Oliver Dr., 40880 Ratingen (DE); Ansorge-Schumacher, Marion Dr., 52159 Roetgen (DE); Wiemann, Lars, 10559 Berlin (DE); Ferenz, Michael, Dr., 45147 Essen (DE); Naumann, Matthias, Dr., Greensboro, NC 27407 (US)

(56) Entgegenhaltungen:
- EP-A1- 2 011 865
- EP-A2- 0 562 371
- DE-A1- 4 019 249
- HOYOS P ET AL: "Highly efficient one pot dynamic kinetic resolution of benzoins with entrapped Pseudomonas stutzeri lipase" JOURNAL OF MOLECULAR CATALYSIS. B, ENZYMATIC, ELSEVIER, AMSTERDAM, NL, Bd. 52-53, 1. Juni 2008 (2008-06-01), Seiten 133-139, XP022586929 ISSN: 1381-1177 [gefunden am 2007-10-25]
- GILL I ET AL: "Bioencapsulation within synthetic polymers (Part 2): non-sol-gel protein-polymer biocomposites" TRENDS IN BIOTECHNOLOGY, ELSEVIER PUBLICATIONS, CAMBRIDGE, GB, Bd. 18, Nr. 11, 1. November 2000 (2000-11-01), Seiten 469-479, XP004237210 ISSN: 0167-7799

## Beschreibung

Die Erfindung betrifft neuartige Enzympräparate zur Verwendung als Biokatalysatoren.

Mikroorganismen und isolierte Enzyme finden verbreiteten Einsatz als Katalysator in der chemischen Industrie oder in der Lebensmittelherstellung. Eine Übersicht bietet beispielsweise: A. Liese, K. Seelbach, C. Wandrey, Indutrial Biotransformations, Wiley-VCH: 2006, Weinheim, Deutschland.

Um einen ökonomischen Einsatz solcher Biokatalysatoren zu gewährleisten, müssen einige Bedingungen erfüllt sein: Der Biokatalysator muss unter den Reaktionsbedingungen hinreichend lange aktiv sein, er sollte nach Ende der Reaktion leicht abtrennbar und möglichst oft wieder verwendbar sein. Idealerweise sollten diese Bedingungen für eine möglichst große Breite an Reaktionsbedingungen (z. B. Temperaturbereich, Art der verwendeten Lösungsmittel, Drücke, etc.) erfüllt sein, um einen möglichst universellen Katalysator bereit zu stellen.

Um diese Bedingungen zu erfüllen, ist es üblicherweise notwendig, die verwendeten Enzyme oder Enzyme enthaltenden Mikroorganismen zu immobilisieren.

Häufig werden die Enzyme oder Enzyme enthaltenden Mikroorganismen auf Trägern nichtkovalent immobilisiert, als Träger werden häufig Ionenaustauscherharze oder Polymerpartikel eingesetzt, die über geeignete Korngrößenverteilungen verfügen. Beispiele hierzu sind die Marktprodukte Novozym 435, Lipozym RM IM oder Lipozym TL IM der Firma Novozymes A/S, Bagsvaerd, Dänemark oder Amano PS, der Firma Amano, Japan. Bei diesen Beispielen handelt es sich um immobilisierte Lipasen, die breite Verwendung finden, da solche Immobilisate auch technisch nutzbare Aktivitäten in nichtwässrigen Systemen, also solchen, die keine oder nur organische Lösungsmittel enthalten, zeigen, wie z. B. in J. Chem. Soc., Chem. Comm. 1989, 934-935 beschrieben.

Die Patentanmeldung DE 10 2007 031689.7 beschreibt die diversen Nachteile der heute verfügbaren Immobilisierungstechnologien, vor allem in Hinblick auf Aktivität und Stabilität.

Gleichzeitig lehrt diese Anmeldung eine neue Klasse von Enzympräparaten sowie ein Verfahren zur ihrer Herstellung, die einen großen Teil dieser Nachteile überwindet. Im Wesentlichen wird beschrieben, dass stabile und hochaktive Enzympräparate dadurch erhalten werden können, dass zunächst Enzyme oder Enzyme enthaltende Mikroorganismen auf einen geeigneten Träger adsorbiert werden und anschließend dieses Immobilisat mit einem Siliconpolymer versehen wird, welches durch eine Hydrosilylierungsreaktion zugänglich ist.

In den Ausführungsbeispielen dieser Anmeldung werden als Bausteine für die Hydrosilylierung neben SiH-Siloxanen verschiedener Topologien allerdings nur mit endständig ungesättigten organischen Resten versehene Siloxane beschrieben.

Zur Feineinstellung der Eigenschaften der so gewonnenen Enzympräparate wäre es allerdings wünschenswert, neben Siloxanketten auch andere Bausteine verwenden zu können. Dem Fachmann ist bekannt, dass Siliconverbindungen besondere Eigenschaften haben, zum Beispiel die eingeschränkte Mischbarkeit mit anderen Substanzklassen, wie zum Beispiel vielen oleochemischen Derivaten oder Wasser.

Es besteht daher weiterhin Bedarf an Methoden zur Enzymimmobilisierung, welche die Nachteile des Standes der Technik überwinden, um bisher nicht realisierbare biokatalytische Verfahren umzusetzen.

Aufgabe der vorliegenden Erfindung war daher die Bereitstellung von Enzympräparaten, die einen oder mehrere der Nachteile der Präparate des Standes der Technik nicht aufweisen. Insbesondere sollten Enzympräparate bereitgestellt werden, die eine vergleichbare Stabilität und Aktivität wie die mit reinen Siliconpolymeren beschichteten Enzympräparate, gleichzeitig aber auch andere Strukturelemente aufweisen.

Weitere nicht explizit genannte Aufgaben ergeben sich aus dem Kontext der nachfolgenden Beschreibung, der Beispiele sowie der Ansprüche.

Überraschenderweise wurde gefunden, dass diese Aufgabe durch Enzympräparate gelöst wird, die durch Immobilisieren von Enzymen oder Enzyme enthaltenden Mikroorganismen auf einem inerten Träger und anschließendes Versehen mit einer durch Hydrosilylierung Si-H-funktioneller Polysiloxane gewonnenen Polyethersiloxanbeschichtung erhalten werden, wobei als olefinische Bausteine mindestens eine terminal ungesättigte Gruppe tragende Polyether und gegebenenfalls zusätzlich Siloxane, die endständig ungesättigte organischen Reste tragen, eingesetzt werden, mit der Maßgabe, dass mindestens ein olefinischer Baustein enthalten ist, der mindestens zwei terminal ungesättigte Gruppen aufweist.

Gegenstand der vorliegenden Erfindung sind daher Enzympräparate, die dadurch erhältlich sind, dass Enzymimmobilisate, die Enzyme oder Enzyme enthaltende Mikroorganismen auf einen inerten Träger immobilisiert aufweisen, mit einer durch Hydrosilylierung Si-H-funktioneller Polysiloxane unter Einsatz von mindestens eine terminal ungesättigte Gruppe tragenden Polyethern gewonnenen Polyethersiloxanbeschichtung versehen werden sowie deren Verwendung als industrieller Biokatalysator.

Außerdem ist Gegenstand der vorliegenden Erfindung ein Verfahren zur Herstellung der erfindungsgemäßen Enzympräparate, welches dadurch gekennzeichnet ist, dass Enzymimmobilisate, die Enzyme oder Enzyme enthaltende Mikroorganismen auf einen inerten Träger immobilisiert aufweisen, mit einer durch Hydrosilylierung Si-H-funktioneller Polysiloxane unter Einsatz von mindestens eine terminal ungesättigte Gruppe tragenden Polyethern gewonnenen Polyethersiloxanbeschichtung versehen werden.

Die erfindungsgemäßen Enzympräparate haben den Vorteil, dass sie eine hohe Stabilität gegenüber mechanischen Kräften und gegenüber Desorption aufweisen. Trotz dieser verbesserten Eigenschaften weisen die erfindungsgemäßen Enzympräparate spezifische Aktivitäten in verschiedenen wässrigen (z. B. in der Hydrolyse von Tributyrin) und nichtwässrigen Reaktionsmischungen (z. B. in der lösungsmittelfreien Synthese von Propyllaurat) auf, die hoch genug sind, um einen technischen Einsatz zu ermöglichen.

Die erfindungsgemäßen Enzympräparate sowie ein Verfahren zu deren Herstellung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z. B. organomodifizierte Polysiloxane, beschrieben, die verschiedene Einheiten mehrfach aufweisen können, so können diese statistisch verteil t (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen.

Die erfindungsgemäßen Enzympräparate zeichnen sich dadurch aus, dass sie erhältlich sind, indem Enzymimmobilisate, die Enzyme oder Enzyme enthaltende Mikroorganismen auf einen inerten Träger immobilisiert aufweisen, mit einer Siliconbeschichtung versehen werden, **die durch** Hydrosilylierung Si-H-funktioneller Polysiloxane gewonnen wird, wobei als olefinische Bausteine mindestens eine terminal ungesättigte Gruppe tragende Polyether und gegebenenfalls zusätzlich Siloxane, die endständig ungesättigte organische Reste tragen, eingesetzt werden, mit der Maßgabe, dass mindestens ein olefinischer Baustein enthalten ist, der mindestens zwei terminal ungesättigte Gruppen aufweist.

Zur Herstellung der Enzymimmobilisate können ganze Zellen, ruhende Zellen, gereinigte Enzyme oder Zellextrakte, die die entsprechenden Enzyme enthalten oder Mischungen davon eingesetzt werden. Bevorzugt eingesetzt werden hydrolytische Enzyme, z. B. Lipasen, Esterasen oder Proteasen, wie beispielsweise Lipasen aus Candida rugosa, Candida antarctica, Pseudomonas sp., Thermomyces lanugiosus, Schweinepankreas, Mucor miehei, Alcaligenes sp., Cholesterolesterase aus Candida rugosa, Esterase aus der Schweineleber, besonders bevorzugt Lipasen, eingesetzt. Demgemäß weisen die Enzymimmobilisate vorzugsweise Enzyme aus der Klasse der Hydrolasen, bevorzugt Lipasen, auf.

Als inerte Träger können inerte organische oder anorganische Träger verwendet werden. Vorzugsweise werden als inerte Träger solche partikulären Träger verwendet bzw. sind im Enzymimmobilisat vorhanden, die eine Teilchengrößenverteilung aufweisen, bei der mindestens 90% der Teilchen eine Teilchengröße von 10 bis 5000 µm, bevorzugt von 50 µm bis 2000 µm aufweisen. Als organische Träger können insbesondere solche eingesetzt werden, die Polyacrylat, Polymethacrylat, Polyvinylstyrol, Styrol-Divinylbenzolcopolymere, Polypropylen, Polyethylen, Polyethylenperepthalat, PTFE und/oder andere Polymere aufweisen oder aus diesen bestehen. Als Trägermaterial können, in Abhängigkeit von dem zu immobilisierenden Enzym, insbesondere saure oder basische Ionenaustauscherharze eingesetzt werden, beispielsweise Duolite A568, Duolite XAD 761, Duolite XAD 1180, Duolite XAD 7HP, Amberlite IR 120, Amberlite IR 400, Amberlite CG 50, Amberlyst 15 (alles Produkte der Fa. Rohm und Haas) oder Lewatit CNP 105 und Lewatit VP OC 1600 (Produkte der Fa. Lanxess, Leverkusen, Deutschland). Als anorganische Träger können aus dem Stand der Technik bekannte, oxidische und/oder keramische Träger eingesetzt werden. Insbesondere können als anorganische Träger beispielsweise Celite, Zeoliten, Silica (Kieselsäure), Controlled-Pore Glas (CPG) oder andere Träger, wie sie zum Beispiel in L. Cao, "Carrier-bound Immobilized Enzymes: Principles, Application and Design", Wiley-VCH: 2005, Weinheim, Deutschland, beschrieben sind, eingesetzt werden. Besonders bevorzugt bestehen die im Enzymimmobilisat vorhandenen inerten Träger bzw. die zur Herstellung der Enzymimmobilisate verwendeten inerten Träger aus Kieselsäure, Polyvinylstyrol, Polymethacrylat oder Polyacrylat.

Die Immobilisierung auf den Partikeln kann erfindungsgemäß kovalent oder nichtkovalent, bevorzugt nichtkovalent erfolgen. Für die nichtkovalente Immobilisierung kann der Träger z. B. mit einer wässrigen Enzymlösung, die gegebenenfalls weitere Bestandteile, wie z. B. anorganische Salze oder Detergenzien, enthalten kann, inkubiert bzw. imprägniert werden. Diese Inkubation/Imprägnierung kann z. B. bei Temperaturen zwischen 0°C und 50°C, bevorzugt zwischen 0°C und 40°C durchgeführt werden. Vorzugsweise erfolgt die Inkubation/Imprägnierung über einen Zeitraum von wenigen Minuten bis zu einigen Stunden. Der Fortschritt der Inkubation kann durch Bestimmung der Konzentration des Enzyms in der Lösung mit den gängigen Verfahren zur Proteinbestimmung erfolgen. Nach Erreichen des gewünschten Immobilisierungsgrads kann der Träger vorzugsweise mit Wasser gewaschen und, wenn gewünscht, getrocknet werden. Ein so erhaltenes Enzymimmobilisat kann anschließend erfindungsgemäß mit einer Polyethersiliconbeschichtung versehen werden.

Erfindungsgemäß können aber auch Enzymimmobilisate eingesetzt werden, die kommerziell erhältlich sind, beispielsweise Novozym 435, Lipozym RM IM oder Lipozym TL IM der Firma Novozymes A/S, Bagsvaerd, Dänemark oder Amano PS, der Firma Amano, Japan.

Die Polyethersiloxanbeschichtung wird erfindungsgemäß durch Hydrosilylierung erhalten. Dazu werden vorzugsweise Si-H-funktionelle Polysiloxane in Gegenwart von Katalysatoren, vorzugsweise von Übergangsmetallkatalysatoren, mit Polyethern, die mindestens eine terminal ungesättigte Gruppe tragen, umgesetzt.

Als Si-H-funktionelle Polysiloxane werden vorzugsweise Si-H-Polysiloxane der allgemeinen Formel (I) eingesetzt,

MₐD_{b}D'_{c}T_{d}Qₑ (I)

wobei
- M =: [R¹₂R^{2a}SiO_{1/2}]
- D =: [R¹₂SiO_{2/2}]
- D' =: [R¹R^{2b}SiO_{2/2}]
- T =: [R¹SiO_{3/2}]
- Q =: [SiO_{4/2}]
- N¹ =: a + b + c + d + e = 3 bis 850, vorzugsweise 6 bis 160,
- a =: 2 bis 10, vorzugsweise 2 bis 4, insbesondere 2,
- b =: 1 bis 800, vorzugsweise 2 bis 150,
- c =: 0 bis 400, vorzugsweise 2 bis 75,
- d =: 0 bis 10, vorzugsweise 0,
- e =: 0 bis 10, vorzugsweise 0,
- R¹: unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C- Atomen, Arylreste mit 6 bis 30 C-Atomen, vorzugsweise Alkylgruppen mit 1 bis 4 C-Atomen oder Phenyl, insbesondere Methyl,
- R^{2a}: unabhängig voneinander Wasserstoff oder R¹ und
- R^{2b}: unabhängig voneinander Wasserstoff oder R¹, sind.

Bevorzugt wird als Si-H-funktionelles Polysiloxan ein Si-H-Polysiloxan der allgemeinen Formel (I) eingesetzt,
wobei
- N¹ =: a + b + c + d + e = 6 bis 160,
- a =: 2,
- b =: 2 bis 150,
- c =: 2 bis 75,
- d =: 0 und
- e =: 0, ist.

Es ist dem Fachmann geläufig, dass die Verbindungen in Form eines Gemisches mit einer im Wesentlichen durch statistische Gesetze geregelten Verteilung vorliegen bzw. vorliegen können. Die Werte für die Indices a, b, c, d und e stellen deshalb üblicherweise Mittelwerte dar.

Erfindungsgemäß werden als mindestens eine terminal ungesättigte Gruppe tragende Polyether solche der allgemeinen Formel (II): 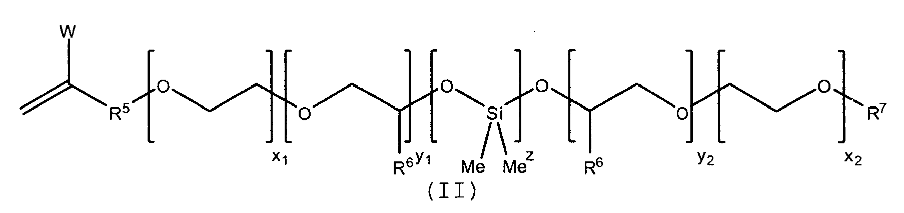 eingesetzt, wobei
- N² =: x₁ + x₂ + y₁ + y₂ = 1 bis 500, vorzugsweise 2 bis 200, insbesondere 3 bis 100,
- x₁ =: 0 bis 200, vorzugsweise 1 bis 200, insbesondere 3 bis 100,
- x₂ =: 0 bis 100, vorzugsweise 1 bis 100, insbesondere 3 bis 100,
- y₁ =: 0 bis 200, vorzugsweise 1 bis 200, insbesondere 3 bis 100,
- y₂ =: 0 bis 100, vorzugsweise 1 bis 200, insbesondere 3 bis 100,
- z =: 0 bis 500, vorzugsweise 0 bis 100,
- W =: Wasserstoff oder Methyl, bevorzugt Wasserstoff,
- R⁵: ein gegebenenfalls verzweigter, gegebenenfalls substituierter, gegebenenfalls Doppelbindungen tragender Alkylenrest mit 1 bis 30 C-Atomen,
- R⁶: unabhängig voneinander ein Rest ausgewählt aus der Gruppe umfassend Methyl, Ethyl oder Phenyl, bevorzugt Methyl und
- R⁷: ein gegebenenfalls verzweigter, gegebenenfalls substituierter, gegebenenfalls Doppelbindungen enthaltender Alkylrest oder Carboxylrest mit 1 bis 30 C-Atomen,
ist, mit der Maßgabe, dass wenn z = 0, x₂ = y₂ = 0 ist.

Es ist dem Fachmann geläufig, dass die Verbindungen in Form eines Gemisches mit einer im Wesentlichen durch statistische Gesetze geregelten Verteilung vorliegen bzw. vorliegen können. Die Werte für die Indices x₁, x₂, y₁ und y₂ stellen deshalb üblicherweise Mittelwerte dar.

Es ist bevorzugt, dass ein Polyether der allgemeinen Formel (II) eingesetzt wird, wobei der Polyether ausschließlich aus Ethylenglycolbausteinen besteht, d. h. y₁ = y₂ = 0; gleichermaßen bevorzugt wird ein Polyether der allgemeinen Formel (II) eingesetzt, wobei der Polyether frei von Ethylenglycolbausteinen ist, d. h. x₁ = x₂ = 0.

Es ist weiterhin bevorzugt, dass ein Polyether der allgemeinen Formel (II) eingesetzt wird, wobei x₁ + x₂ und y₁ + y₂ jeweils größer als 1, besonders bevorzugt jeweils größer als 3 sind.

Es kann vorteilhaft sein, wenn Polyether der allgemeinen Formel (II) eingesetzt werden, bei denen z = 0 ist.

In einer bevorzugten Ausführungsform des Einsatzes von Polyethern als olefinischer Reaktionspartner wird ein Polyether der allgemeinen Formel (II) mit z = 0 eingesetzt, wobei
- R⁵: ein Alkylenrest mit 1 bis 30 C-Atomen, bevorzugt mit 1 bis 9 C-Atomen, besonders bevorzugt mit 1 bis 4 C- Atomen, insbesondere Methylen und
- R⁷: ein gegebenenfalls verzweigter, terminal ungesättigter Alkylrest mit 3 bis 30 C-Atomen, bevorzugt mit 3 bis 11 C-Atomen, besonders bevorzugt mit 3 bis 6 C-Atomen, insbesondere Allyl oder Methallyl,
ist.

Es kann aber auch vorteilhaft sein, wenn Polyether der allgemeinen Formel (II) eingesetzt werden, bei denen z > 0 ist.

In einer bevorzugten Ausführungsform des Einsatzes von Polyethern als olefinischer Reaktionspartner wird ein Polyether der allgemeinen Formel (II) eingesetzt, wobei z = 1 bis 500, vorzugsweise 3 bis 200, insbesondere 6 bis 100 ist. Bei dieser Ausführungsform ist es bevorzugt, dass ein Polyether der allgemeinen Formel (II) eingesetzt wird, wobei
- N² =: x₁ + x₂ + y₁ + y₂ - 2 bis 500, vorzugsweise 4 bis 200, insbesondere 6 bis 100,
- R⁵: ein Alkylenrest mit 1 bis 30 C-Atomen, bevorzugt mit 1 bis 9 C-Atomen, besonders bevorzugt mit 1 bis 4 C- Atomen, insbesondere Methylen und
- R⁷: ein gegebenenfalls verzweigter, terminal ungesättigter Alkylrest mit 3 bis 30 C-Atomen, bevorzugt mit 3 bis 11 C-Atomen, besonders bevorzugt mit 3 bis 6 C-Atomen, insbesondere Allyl oder Methallyl,
ist.

Erfindungsgemäß können auch Mischungen der Polyether, die mindestens eine terminal ungesättigte Gruppe tragen, als olefinische Reaktionspartner eingesetzt werden.

Bevorzugt werden als Polysiloxane, die endständig ungesättigte organische Reste tragen, Polysiloxane der allgemeinen Formel (III):

MₘDₙD' oTₚQ_{q} (III)

eingesetzt, wobei
- M =: [R³₂R⁴SiO_{1/2}]
- D =: [R³₂SiO_{2/2}]
- D' =: [R³R⁴SiO_{2/2}]
- T =: [R³SiO_{3/2}]
- Q =: [SiO_{4/2}]
- N² =: m + n + o + p + q = 3 bis 1000, vorzugsweise 10 bis 600,
- m =: 2 bis 10, vorzugsweise 2 bis 4, insbesondere 2,
- n =: 1 bis 800, vorzugsweise 2 bis 600,
- o =: 0 bis 20, vorzugsweise 0 bis 10, bevorzugt 0,
- p =: 0 bis 10, vorzugsweise 0,
- q =: 0 bis 10, vorzugsweise 0,

- R³: unabhängig voneinander gleich oder verschieden und ausgewählt sind aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl und
- R⁴: unabhängig voneinander ein terminal ungesättigter Alkylrest, bevorzugt Vinyl, oder ein Alkoxylrest, vorzugsweise mit 3 bis 20 C-Atomen, oder R₃, sind.

Als endständige Polysiloxane, die endständig ungesättigte organische Reste tragen, werden bevorzugt Polysiloxane der allgemeinen Formel (III) eingesetzt, wobei
- N² =: m + n + o + p + q = 10 bis 600,
- M =: 2,
- n =: 2 bis 600,
- o =: 0,
- p =: 0,
- q =: 0 und
- R⁴: unabhängig voneinander ein terminal ungesättigter Alkylrest, bevorzugt Vinyl, ist.

Es ist dem Fachmann geläufig, dass die Verbindungen der Formel (III) in Form eines Gemisches mit einer im Wesentlichen durch statistische Gesetze geregelten Verteilung vorliegen bzw. vorliegen können. Die Werte für die Indices m, n, o, p und q stellen deshalb üblicherweise Mittelwerte dar.

Wenn der Rest R⁷ des eingesetzten Polyethers keine terminale Doppelbindung aufweist, werden Verbindungen der allgemeinen Formeln (II) und (III) im molaren Verhältnis von 0,001:1 bis 1:0,001, bevorzugt von 0,05:1 bis 5:1, insbesondere von 0,05:1 bis 1:1 eingesetzt.

Wenn der Rest R⁷ eine terminale Doppelbindung aufweist, können erfindungsgemäß zusätzlich Siloxane, die endständig ungesättigte organische Reste tragen, eingesetzt werden. In diesem Falle werden Verbindungen der allgemeinen Formeln (II) und (III) im molaren Verhältnis von 0,001:1 bis 1:0, bevorzugt von 0,05:1 bis 20:1, insbesondere von 0,10:1 bis 10:1 eingesetzt werden.

Bei dem Einsatz von Polyethern als olefinischer Reaktionspartner zusammen mit einer weiteren olefinischen Komponente der allgemeinen Formel (III) wird bevorzugt ein Polyether der allgemeinen Formel (II) mit z = 0 eingesetzt, wobei
- R⁵: ein Alkylenrest mit 1 bis 30 C-Atomen, bevorzugt mit 1 bis 9 C-Atomen, besonders bevorzugt mit 1 bis 4 C- Atomen, insbesondere Methylen und
- R⁷: ein Alkylrest mit 1 bis 30 C-Atomen, bevorzugt mit 1 bis 9 C-Atomen, besonders bevorzugt mit 1 bis 4 C- Atomen, insbesondere Methyl,
ist.

Die Hydrosilylierung kann nach etablierten Methoden in Gegenwart eines Katalysators durchgeführt werden. Dabei können beispielsweise Katalysatoren verwendet werden, die üblicherweise für Hydrosilylierungen eingesetzt werden, wie beispielsweise Platin-, Rhodium-, Osmium-, Ruthenium-, Palladium-, Iridium-Komplexe oder ähnliche Verbindungen bzw. die entsprechenden reinen Elemente oder deren auf Silica, Aluminiumoxid oder Aktivkohle oder ähnlichen Trägermaterialien immobilisierte Derivate. Bevorzugt wird die Hydrosilylierung in Gegenwart von Pt-Katalysatoren wie Cis-Platin oder Karstedt-Katalysator [Tris(divinyltetramethyldisiloxan)bis-platin] durchgeführt.

Vorzugsweise beträgt die eingesetzte Menge an Katalysator 10⁻⁷ bis 10⁻¹ mol pro mol Olefin bzw. pro mol endständiger Kohlenstoff-Kohlenstoff Doppelbindung, bevorzugt 1 bis 100 ppm. Die Hydrosilylierung wird vorzugsweise bei Temperaturen von 0 und 200 °C, bevorzugt von 20 bis 120 °C, durchgeführt.

Die Hydrosilylierung kann in Gegenwart oder in Abwesenheit von Lösungsmittel durchgeführt werden. Generell sind Lösungsmittel für die Durchführung der Reaktion nicht nötig. Die Reaktion kann jedoch in geeigneten Lösungsmitteln, wie beispielsweise aliphatischen oder aromatischen Kohlenwasserstoffen, cyclischen Oligosiloxanen, Alkoholen oder Estern, durchgeführt werden. Geeignete Lösungsmittel sind insbesondere Cyclohexan oder Toluol.

Erfindungsgemäß werden bezogen auf die Masse des eingesetzten Trägers vorzugsweise 1 bis 500 Massen-%, bevorzugt 10 bis 300 Massen-%, besonders bevorzugt 20 bis 200 Massen-% an Siloxan- und Polyetherkomponenten eingesetzt. Die Siloxan- und Polyetherkomponenten setzen sich insbesondere aus der Summe der Verbindungen der Formel (I), (II) und (III) bzw. aus deren Reaktionsprodukten zusammen.

Die Hydrosilylierung kann unter Verwendung der verschiedensten Verhältnisse der Verbindungen der Formeln (I) zu Verbindungen der Formeln (II), bzw. zu Mischungen von Verbindungen der Formeln (II) und III) durchgeführt werden. Vorzugsweise erfolgt die Hydrosilylierung bei einem molaren Verhältnis bezogen auf die reaktiven Gruppen von 1 : 10 bis 10 zu 1, bevorzugt von 1 : 5 bis 5 : 1, besonders bevorzugt von 1 : 1,5 bis 1,5 : 1 und ganz besonders bevorzugt von 1 : 1,3 bis 1,3 : 1. Durch Wahl der eingesetzten Verbindungen der allgemeinen Formel (I), (II) und ggf. (III) und Veränderung ihrer Mischungsverhältnisse lassen sich die Eigenschaften der Polyethersiliconbeschichtung Maßschneidern in Hinblick auf Durchlässigkeit für Substrate und andere Reaktionseigenschaften. Durch Wahl des Gewichtsverhältnisses von Siliconkomponenten zu Enzymimmobilisaten lassen sich die Schichtdicken der Polyethersiliconbeschichtung variieren und an entsprechende Anforderungen angleichen.

Die erfindungsgemäße Polyethersiloxanbeschichtung, hergestellt durch Hydrosilylierung, kann dadurch erhalten werden, dass die Hydrosilylierung in Gegenwart der Enzymimmobilisate durchgeführt wird. Es ist aber auch möglich dass die Enzymimmobilisate nachträglich mit durch Hydrosilylierung erhaltene Polyethersiloxane versehen werden. Dies kann z. B. so erfolgen, dass die Enzymimmobilisate mit einer Lösung der Siloxane und der Polyether, z. B. einer Lösung der Siloxane und Polyether in einem organischen Lösungsmittel, insbesondere Cyclohexan oder Toluol, behandelt werden. Anschließend kann das Lösungsmittel z. B. durch Abdampfen entfernt werden. Die Konzentration an Siloxanen und Polyethern in einer solchen Lösung beträgt vorzugsweise 10 bis 100 Massen-%, bevorzugt 30 bis 100 Massen-%. Vorzugsweise wird die erfindungsgemäße Polyethersiloxanbeschichtung aber dadurch erhalten, dass die Hydrosilylierung in Gegenwart der Enzymimmobilisate durchgeführt wird.

Die erfindungsgemäßen Enzympräparate werden vorzugsweise hergestellt durch das nachfolgend beschriebene erfindungsgemäße Verfahren. Dieses Verfahren zur Herstellung von Enzympräparaten zeichnet sich dadurch aus, dass Enzymimmobilisate, die Enzyme oder Enzyme enthaltende Mikroorganismen auf einen inerten Träger immobilisiert aufweisen, mit einer durch Hydrosilylierung Si-H-funktioneller Polysiloxane gewonnenen Polyethersiloxanbeschichtung versehen werden, bei denen als olefinische Bausteine mindestens eine terminal ungesättigte Gruppe tragende Polyether und gegebenenfalls zusätzlich Siloxane, die endständig ungesättigte organischen Reste tragen, eingesetzt werden, mit der Maßgabe, dass mindestens ein olefinischer Baustein enthalten ist, der mindestens zwei terminal ungesättigte Gruppen aufweist.

Vorzugsweise wird das erfindungsgemäße Verfahren so durchgeführt, dass die Enzymimmobilisate dadurch mit einer Polyethersiloxanbeschichtung versehen werden, dass die Enzymimmobilisate mit einer Reaktionsmischung, die SiH-funktionelle Polysiloxane, mindestens eine terminal ungesättigte Gruppe tragenden Polyether und gegebenenfalls endständige Kohlenstoff-Kohlenstoff Doppelbindungen enthaltende Polysiloxane sowie einen die Hydrosilylierung katalysierenden Katalysator aufweist, unter Hydrosilylierungsbedingungen in Kontakt gebracht werden.

Vorzugsweise werden in dem erfindungsgemäßen Verfahren die als oben für die erfindungsgemäßen Enzympräparate bevorzugt genannten Komponenten (SiH-funktionelle Polysiloxane, mindestens eine terminal ungesättigte Gruppe tragende Polyether und endständige Kohlenstoff-Kohlenstoff Doppelbindungen enthaltende Polysiloxane) eingesetzt.

Insbesondere kann das Verfahren so durchgeführt werden, dass in Gegenwart von Enzymimmobilisaten, die Enzyme oder Enzyme enthaltende Mikroorganismen auf einen inerten Träger immobilisiert aufweisen, eine Hydrosilylierungsreaktion durchgeführt wird. Durch das bei der Hydrosilylierung entstehende Polyethersiloxan kann das Enzymimmobilisat mit einer Polyethersiloxanbeschichtung versehen werden.
Die Hydrosilylierung kann auf eine dem Fachmann bekannte Weise durchgeführt werden. Vorzugsweise wird die Hydrosilylierung unter Verwendung der oben genannten Parameter/Einsatzstoffe/Katalysatoren durchgeführt.

In einer bevorzugten Ausführungsform des erfindungsgemäßen Verfahrens wird eine bestimmte Menge Enzymimmobilisat mit einer Mischung (Reaktionsmischung) der Siliconreagentien (Verbindungen der Formeln (I), (II) und gegebenenfalls (III) plus Katalysator) versetzt, beispielsweise durch Zugabe von einer Mischung enthaltend Verbindungen der allgemeinen Formeln (I), (II) und gegebenenfalls (III) sowie Karstedt-Katalysator. So kann z. B. zu 1 g eines Enzymimmobilisats eine Mischung von Verbindungen der Formel (I), (II) und (III) im molaren Mischungsverhältnis von 1:0,6:0,6, sowie Karstedt-Kat, bspw. 50 ppm bezogen auf die vorhandene Menge an Siliconkomponenten, gegeben werden. Zwecks Optimierung der Beschichtungseigenschaften kann es vorteilhaft sein, die Siliconkomponenten inklusive des Katalysators vor der Zugabe in einem Lösungsmittel, beispielsweise Cyclohexan, Toluol oder einem anderen organischen Lösungsmitteln zu lösen und dann die Lösung zu dem Enzymimmobilisat zu geben. Wird beispielsweise Toluol als Lösungmittel eingesetzt, hat es sich als vorteilhaft erwiesen, nach Zugabe der Lösung zu den Enzymimmobilisaten diese Mischung für ca. 30 bis 45 min. stark zu dispergieren, z. B. durch einen Vortexer (Ika, Stufe 9), bis ein Gros des Toluols abgedampft ist. Anschließend werden die erhaltenen Enzympräparate im Trockenschrank bei 50°C beispielsweise für 12 Stunden getrocknet, bzw. gehärtet. Durch Veränderung der Mischungsverhältnisse der Verbindungen der allgemeinen Formel (I), (II) und gegebenenfalls (III) können die Eigenschaften des Polyethersiliconcoatings problemlos variiert und an entsprechende Anforderungen angeglichen werden.

Eine weitere Ausführungsform des erfindungsgemäßen Verfahrens unterscheidet sich von der vorgenannten Ausführungsform dadurch, dass die zu beschichtenden Enzymimmobilisate in die gewünschte Reaktionsmischung eingetaucht, anschließend aus der Reaktionsmischung entfernt und getrocknet werden. Das Entfernen kann z. B. unter Verwendung eines Siebes, das die Enzymimmobilisat-Partikel zurückhält, erfolgen. Die Eintauchdauer beträgt vorzugsweise 1 bis 10 Minuten. Das Trocknen kann in einem herkömmlichen Trockenschrank erfolgen. Vorzugsweise erfolgt die Trocknung/Härtung bei einer Temperatur von 20°C bis 80°C, bevorzugt bei 40°C bis 60°C, besonders bevorzugt bei ca. 50°C.

In einer weiteren Ausführungsform des erfindungsgemäßen Verfahrens, die insbesondere zur Durchführung im technischen Maßstab geeignet ist, wird die Hydrosilylierung unter Verwendung einer Pelletiertellereinheit (bspw. der Fa. Erweka oder Eirich) durchgeführt. Dabei wird eine definierte Menge an Enzymimmobilisat- **Partikeln in die so** genannte Tellereinheit gegeben und gerührt. Anschließend wird entweder die Mischung, enthaltend Verbindungen der Formeln (I), (II) und gegebenenfalls (III), sowie Katalysator sowie ggf. Lösungsmittel zugegeben oder aber vorzugsweise wird bzw. werden, unter Verwendung einer Zweistoffdüse (z. B. der Firma Schlick oder andere) unter Druck (bspw. Stickstoff oder synthetische Luft) die Mischung bzw. die Komponenten in Form eines feinen Tröpfchennebels appliziert, um eine möglichst homogene Verteilung auf den Partikeln zu gewährleisten. Die Partikel werden nach einer längeren Beschichtungszeit, wie oben beschrieben entnommen und für einige Stunden bei einer Temperatur von 20°C bis 80°C, bevorzugt von 40°C bis 60°C, besonders bevorzugt von 50°C im Trockenschrank getrocknet, bzw. gehärtet, und können anschließend bis zur weiteren Verwendung bei Raumtemperatur gelagert werden.

In einer weiteren Ausführungsform können die Partikel in einem Wirbelschichtreaktor (bspw. Fa. Glatt) generiert werden, in dem Partikel und die Reaktionsmischung unter starker Dispergierung in entsprechenden Mischungsverhältnissen appliziert werden. Erfindungsgemäß kann dies sowohl im sogenannten *top-down-* als auch im sogenannten *bottom*-*up*-Verfahren (auch bekannt als Wurster-Verfahren) durchgeführt werden.

Die erfindungsgemäßen Enzympräparate können z. B. als Biokatalysatoren, insbesondere als industrielle Biokatalysatoren verwendet werden.

Die nachfolgenden Beispiele sollen die vorliegende Erfindung näher erläutern, ohne den Schutzbereich einzuschränken, der sich aus der Beschreibung und den Patentansprüchen ergibt.

### Beispiele:

### Materialien und Methoden:

Novozym 435 (NZ435) ist ein kommerzielles Enzymimmobilisat der Firma Novozymes A/S, Bagsvaerd/Dänemark, im Detail eine auf einem Polymethacrylat durch Adsorption immobilisierte Lipase B aus *C. antarctica.*

### Hydrolytische Aktivität (Tributyrinhydrolyse in wässrigem Medium):

Die hydrolytische Aktivität wurde mit der sogenannten pH-Stat.-Methode bestimmt, hierbei wird die in der Hydrolyse freigesetzte Säure gegen eine Base titriert, so dass der pH-Wert der Lösung konstant gehalten wird. Aus der Zeitabhängigkeit des Verbrauchs an Base lässt sich die freigesetzte Säure, und damit die Enzymaktivität quantifizieren. Exemplarische Durchführung: Es wurden 10 bis 20 mg katalytisch aktive Partikel zu 25 mL Tris-HCl-Puffer (1 mM, pH 7,5; enthält zusätzlich 0,1 mM NaCl und CaCl₂) und 500 µL Tributyrin gegeben. Die hydrolytische Aktivität wurde am Autotitrator (Tritroline alpha, Fa. Schott) über die Menge der zutitrierten Base (50 mM NaOH) quantifiziert.

### Syntheseaktivität in PLU Units (Propyllauratsyntheseaktivität in lösungsmittelfreiem System):

Es wurden 10 mg katalytisch aktive Partikel in 5 mL äquimolare Substratlösung (Laurinsäure und 1-Propanol) gegeben und bei 60°C schüttelnd bzw. rührend inkubiert. Proben (V_{probe}: 50 µL) wurden über 25 min alle 5 min gezogen und in 950 µL Decan (Interner Standard: 4 mM Dodecan) überführt. Die Bestimmung der PLUs erfolgte anhand der initialen Produktbildungsraten. Der Nachweis von Propyllaurat (Retentionszeit: 9.791 min) erfolgte gaschromatografisch (Shimadzu 2010, Säule BTX Fa. SGE; Länge 25 m, I.D. 0,22 µm; Film: 0,25 µM; Detektortyp: FID bei 300°C; Injektortemperatur 275°C und Injektionsvolumen 1 µL, Splitratio 35.0; Trägergasdruck (Helium) 150 kpa; Temperaturprogramm: Anfangstemperatur 60°C hold 1,5 min, Temperaturanstieg 20°C/min, Endtemperatur 250°C hold 2,5 min).

### Vergleichsbeispiel 1:

### Bestimmung der Desorptionsstabilität konventioneller Enzymimmobilisate

Zwecks Bestimmung der Desorptionsstabiliät der Partikel unter harschen Reaktionsbedingungen wurden Fraktionen von 20,0 mg NZ435 in 20 mL MeCN/H₂O(1:1, v/v)-Lösung bei 45°C für 30 min. geschüttelt (im Nachfolgenden "Inkubiert"). Die Partikel wurden über einen Faltenfilter zurückgewonnen und mit 100 mL H₂O gewaschen und für 12 h bei 50°C getrocknet, um anschließend nach oben beschriebenen Schema die hydrolytische Aktivität und die Syntheseaktivität in PLU zu bestimmen und mit den Ergebnissen von nativem NZ435 aus der verwendeten Charge zu vergleichen. Die Ergebnisse können auch zur leichter verständlichen Darstellung prozentual zu der Ausgangsaktivität (im Nachfolgenden "Recovery rate") angegeben werden. Die Ergebnisse sind Tabelle 1 zu entnehmen.

**Tabelle 1:**

| Messergebnisse Vergleichsbeispiel 1 | | | | |
|---|---|---|---|---|
| Enzyme | Syntheseakt. vor Inkubation [PLU/g] | Syntheseakt. nach Inkubation [PLU/g](Recovery rate) | Hydrolytische Aktivität vor Inkubation [U/mg] | Hydrolytische Aktivität nach Inkubation [U/mg](Recovery rate) |
| NZ435 nativ | 4400 | 147 (3,3%) | 1,54 | 0,10 (6,5%) |

### Beispiel 1:

### Herstellung eines stabilen Enzympräparates

### Exemplarische Herstellung:

1 g NZ435-Partikel wurden in einer Metallschale mit der Reaktionsmischung, bestehend aus verschiedenen Zusammensetzungen von Verbindungen der allgemeinen Formeln (I, (II) und (III) (Zusammensetzung siehe Tabelle 2, die Komponenten der allgemeinen Formeln (I) und (III) wurden nach dem Fachmann geläufigen Verfahren, wie zum Beispiel in US 7,196,153 B2 beschrieben, durch Equilibrierung hergestellt; die Monoallylpolyether der allg. Formel (II) (R⁷ = Methyl) wurden nach dem Fachmann geläufigen Verfahren, wie zum Beispiel in EP 0427088 A2 oder DE 2800710 A1 beschrieben, hergestellt, die Diallylpolyether der allg. Formel (II) (R⁷ = Allyl) wurden von der Fa. Clariant bezogen, sowie Karstedt-Katalysator (Syloff 4000, Produkt von Dow Corning, USA), versetzt. Die Siliconkomponenten inklusive des Katalysators wurden jeweils vor der Applikation in 10 mL Toluol gelöst und dann in die Metallschale zu den Partikeln gegeben. Nach der Zugabe wurde unmittelbar mittels Vortexer (Ika, Stufe 9) für 30 bis 45 min. stark dispergiert bis ein Gros des Toluols abgedampft war. Anschließend wurden die Partikel für etwa 12 h im Trockenschrank bei 50°C getrocknet. Die Ergebnisse sind in Tabelle 2 dargestellt. Die Aktivitäten der beschichteten Partikel sind sowohl bezogen auf die Gewichtseinheit an beschichtetem Partikel als auch zur besseren Vergleichbarkeit auf die eingesetzte Menge nativem NZ435 bezogen. Schließlich sind letztere Werte zusätzlich im prozentualen Verhältnis zur Aktivität des nativen NZ435 (im Nachfolgenden "Aktivitätsausbeute") dargestellt.

**Tabelle 2: Zusammensetzung der verschiedenen beschichteten Partikel**

| Nr. | Einwaage NZ435 | Komponente der allg. Formel (I); c = d = 0; R¹ = R^{2a} = Methyl, R^{2b} = H | Komponente der allg. Formel (II); W = H; R⁵ -CH₂-; R⁶ = Methyl; | Komp. der allg. Formel (III); b = c = d = 0; R³ = Methyl, R⁴ = Vinyl | Anteil NZ435 [%] | Hydrolyseakt. [U/mg Immob. (U/mg NZ435; Aktivitätsausbeute)] | Syntheseakt. [PLU/g Immob. (PLU/g NZ435; Aktivitätsausbeute)] |
|---|---|---|---|---|---|---|---|
| NZ435¹ | 1 g | - | - | - | 100 | 1,54 | 4400 (4400) |
| i | 1 g | a = 43, b = 5 490 mg | x₁ = 25, x₂ = y₁ = y₂ = z = 0, R⁷ = Allyl; 71,4 mgL | a = 98 438,6 mg | 50 | 0,50 (1,00; 65%) | 1803 (3606; 82%) |
| ii | 1 g | a = 43, b = 5 735 mg | x₁ = 25, x₂ = y₁ = y₂ = 0, R⁷ = Allyl; 107,1 mg | a = 98 657,9 mg | 40 | 0,48 (1,20; 78%) | 1504 (3759; 85%) |
| iii | 1 g | a = 43, b = 5 514,1 mg | x₁ = 25, x₂ = y₁ = y₂ = z = 0, R⁷ = Allyl; 38,9 mg | a = 98 447 mg | 50 | 0,47 (0,99; 55%) | 1767 (3534; 80%) |
| iv | 1 g | a = 43, b = 5 771,3 mg | x₁ = 25, x₂ = y₁ = y₂ = z = 0, R⁷ = Allyl; 58,4 mg | a = 98 670,2 mg | 40 | 0,39 (0,98; 64%) | 1592 (3980; 90%) |
| v | 2 g | a = 43, b = 5 450 mg | x₁ = 23, y₁ = 5, x₂ = y₂ = z = 0, R⁷=Methyl; 190 mg | a = 98 2360 mg | 40 | 0,47 (1,20; 78%) | 1540 (3850; 88%) |
| vi | 2 g | a = 43, b = 5 470 mg | x₁ = 23, y₁ = 5, x₂ = y₂ = z = 0, R⁷ = Methyl; 100 mg | a = 98 2430 mg | 40 | 0,47 (1,20; 78%) | 1875 (4687; 106%) |
| vii | 2 g | a = 43, b = 5 420 mg | x₁ = x₂ = 2, y₁ = y₂ = 8, z = 15, R⁷ = Allyl; 340 mg | a = 98 2240 mg | 40 | 0,41 (1,03; 67%) | 1771 (4428; 100%) |
| viii | 2 g | a = 43, b = 5 410 mg | x₁ = x₂ = 2, y₁ = y₂ = 8, z = 15, R⁷ = Allyl; 390 mg | a = 98 2200 mg | 40 | 0,36 (0,90; 58%) | 1710 (4275; 97%) |

| | | | | | | | |
|---|---|---|---|---|---|---|---|
| ¹Daten für natives NZ435 entnommen aus Vergleichsbeispiel 1 | | | | | | | |

Die nach diesem Verfahren hergestellten Partikel weisen gegenüber dem unbehandelten Immobilisat Aktivitätsausbeuten in der Hydrolyse von 55% bis zu 78% (Beispiel 1 i, 1,20 U/mg NZ435 im Vergleich zu 1,54U/mg für unbehandeltes NZ435, wie in Vergleichsbeispiel 1 beschrieben). In der Synthese konnten sehr gute Aktivitätsausbeuten von 80% bis zu 106% erreicht werden.

### Beispiel 2:

### Bestimmung der Desorptionsstabilität stabiler Enzympräparate

Analog zu Vergleichsbeispiel 1 wurden die gewonnenen Partikel aus Beispiel 1 mit Wasser/Acetonitril behandelt und anschließend die Hydrolyseaktivität und die Syntheseaktivität bezogen auf den Gewichtsanteil an NZ435 in dem Präparat bestimmt. Das Ergebnis dieser Bestimmung **kann** Tabelle 3 entnommen werden, analog zu Vergleichsbeispiel 1 wurde auch die recovery rate bestimmt.

**Tabelle 3:**

| Ergebnisse aus Beispiel 2 | | | | | |
|---|---|---|---|---|---|
| | Anteil NZ435 [%] | Hydrolyseakt. vor Inkubation [U/mg NZ435] | Hydrolyseakt. nach Inkubation [U/mg NZ435]; (recovery rate) | Syntheseakt. vor Inkubation [U/mg NZ435] | Syntheseakt. nach Inkubation [U/mg NZ435]; (recovery rate) |
| NZ435¹ | 100 | 1,54 | 0,10; (6,5%) | 3761 | 147; (3,9%) |
| i | 50 | 1,00 | 0,38; (38%) | 3606 | 340; (9,4%) |
| ii | 40 | 1,20 | 0,81; (68%) | 3759 | 2402; (64%) |
| iii | 50 | 0,94 | 0,28; (30%) | 3534 | 240; (6,8%) |
| iv | 40 | 0,98 | 0,58; (59%) | 3980 | 1500; (33%) |
| v | 40 | 1,20 | 0,38; (32%) | 3850 | 425; (11%) |
| vi | 40 | 1,20 | 0,43; (36%) | 4587 | 875; (19%) |
| vii | 40 | 1,03 | 0,50; (51%) | 4428 | 603; (14%) |
| viii | 40 | 0,90 | 0,55; (61%) | 4275 | 695; (16%) |

| | | | | | |
|---|---|---|---|---|---|
| ¹Daten für natives NZ435 entnommen aus Vergleichs beispiel 1 | | | | | |

Während unbehandeltes, natives Enzymimmobilisat nach Inkubation fast keinerlei Hydrolyse- oder Synthese aktivität zeigt , zeigen die polyethersiloxanbeschichteten Partikel Hydrolyseaktivitäten von bis zu 68% der Ausgangsaktivität und Syntheseaktivitäten von bis zu 64 % der Ausgangsaktivität.

## Patentansprüche

1. Enzympräparate, **dadurch** erhältlich, dass Enzymimmobilisate, die Enzyme oder Enzyme enthaltende Mikroorganismen auf einen inerten Träger immobilisiert aufweisen, mit einer durch Hydrosilylierung Si-H-funktioneller Polysiloxane gewonnenen Polyethersiloxanbeschichtung versehen werden, bei denen als olefinische Bausteine mindestens eine terminal ungesättigte Gruppe tragende Polyether und gegebenenfalls zusätzlich Siloxane, die endständig ungesättigte organische Reste tragen, eingesetzt werden, mit der Maßgabe, dass mindestens ein olefinischer Baustein enthalten ist, der mindestens zwei terminal ungesättigte Gruppen aufweist.

2. Enzympräparate nach Anspruch 1, **dadurch gekennzeichnet, dass** die Enzymimmobilisate Enzyme aus der Klasse der Hydrolasen, bevorzugt Lipasen, aufweisen.

3. Enzympräparate nach einem der Ansprüche 1 oder 2, **dadurch gekennzeichnet, dass** die inerten Träger eine Korngrößenverteilung aufweisen, bei der 90% der Teilchen eine Teilchengröße von 10 bis 5000 µm aufweisen.

4. Enzympräparate nach einem der Ansprüche 1 bis 3, **dadurch gekennzeichnet, dass** die eingesetzten inerten Träger aus Kieselsäure, Polyvinylstyrol, Polymethacrylat oder Polyacrylat bestehen.

5. Enzympräparate nach einem der Ansprüche 1 bis 4, **dadurch gekennzeichnet, dass** als Si-H-funktionelle Polysiloxane Si-H-Polysiloxane der allgemeinen Formel (I) MₐD_{b}D'_{c}T_{d}Qₑ (I) eingesetzt werden, wobei
M = [R¹R^{2d}SiO_{1/2}]
D = [R¹₂SiO_{2/2}]
D' = [R¹R^{2b}SiO_{2/2}]
T = [R¹SiO_{3/2}]
Q = [SiO_{4/2}]
N¹ = a + b + c + d + e = 3 bis 850,
a = 2 bis 10,
b = 1 bis 800,
c = 0 bis 400,
d = 0 bis 10,
e = 0 bis 10,
R¹ unabhängig voneinander gleich oder verschieden, ausgewählt aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen,
R^{2a} unabhängig voneinander Wasserstoff oder R¹ und
R^{2b} unabhängig voneinander Wasserstoff oder R¹,
sind.

6. Enzympräparate nach einem der Ansprüche 1 bis 5, **dadurch gekennzeichnet, dass** als Si-H-funktionelle Polysiloxane Si-H-Polysiloxane der allgemeinen Formel (I) eingesetzt werden, wobei
N¹ = a + b + c + d + e = 6 bis 160,
a = 2,
b = 2 bis 150,
c = 2 bis 75,
d = 0 und
e = 0.

7. Enzympräparate nach einem der Ansprüche 1 bis 6, **dadurch gekennzeichnet, dass** als mindestens eine terminal ungesättigte Gruppe tragende Polyether solche der allgemeinen Formel (II): 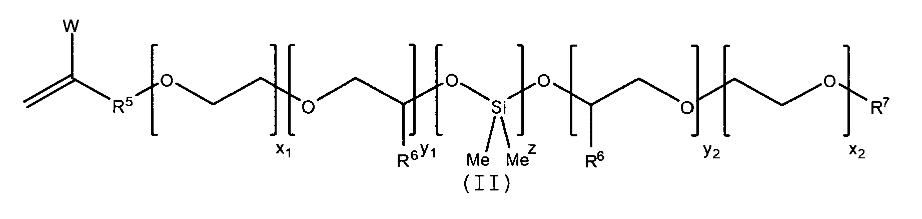 eingesetzt werden, wobei
N² = x₁ + x₂ + y₁ + y₂ = 1 bis 500,
x₁ = 0 bis 200,
x₂ = 0 bis 100,
y₁ = 0 bis 200,
y₂ = 0 bis 100,
z = 0 bis 500,
W = Wasserstoff oder Methyl,
R⁵ ein gegebenenfalls verzweigter, gegebenenfalls substituierter, gegebenenfalls Doppelbindungen tragender Alkylenrest mit 1 bis 30 C-Atomen,
R⁶ unabhängig voneinander ein Rest ausgewählt aus der Gruppe umfassend Methyl, Ethyl oder Phenyl und
R⁷ ein gegebenenfalls verzweigter, gegebenenfalls substituierter, gegebenenfalls Doppelbindungen enthaltender Alkylrest oder Carboxylrest mit 1 bis 30 C-Atomen ist, mit der Maßgabe, dass wenn z = 0, x₂ = y₂ = 0 ist.

8. Enzympräparate nach einem der Ansprüche 1 bis 7, **dadurch gekennzeichnet, dass** als endständige Kohlenstoff-Kohlenstoff Doppelbindungen enthaltende Polysiloxane Polysiloxane der allgemeinen Formel (III) MₘDₙD'ₒTₚQ_{q} (III)
eingesetzt werden, wobei
M = [R³₂R⁴SiO_{1/2}]
D = [R³₂SiO_{2/2}]
D' = [R³R⁴SiO_{2/2}]
T = [R³SiO_{3/2}]
Q = [SiO_{4/2}]
N² = m + n + o + p + q = 3 bis 1000,
m = 2 bis 10,
n = 1 bis 800,
o = 0 bis 20,
p = 0 bis 10,
q = 0 bis 10,
R³ unabhängig voneinander gleich oder verschieden und ausgewählt sind aus der Gruppe umfassend: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen und
R⁴ unabhängig voneinander ein terminal ungesättigter Alkylrest, bevorzugt Vinyl, oder ein Alkoxylrest oder R₃, sind.

9. Enzympräparate nach einem der Ansprüche 1 bis 8, **dadurch gekennzeichnet, dass** als endständige Kohlenstoff-Kohlenstoff-Doppelbindung enthaltende Polysiloxane solche der allgemeinen (Formel III) eingesetzt werden, wobei
N² = m + n + o + p + q = 10 bis 600,
m = 2,
n = 2 bis 600,
o = 0,
p = 0,
q = 0 und
R⁴ unabhängig voneinander ein terminal ungesättigter Alkylrest, sind.

10. Verfahren zur Herstellung von Enzympräparaten, **dadurch gekennzeichnet, dass** Enzymimmobilisate, die Enzyme oder Enzyme enthaltende Mikroorganismen auf einen inerten Träger immobilisiert aufweisen, mit einer durch Hydrosilylierung Si-H-funktioneller Polysiloxane gewonnenen Polyethersiloxanbeschichtung versehen werden, bei denen als olefinische Bausteine mindestens eine terminal ungesättigte Gruppe tragende Polyether und gegebenenfalls zusätzlich Siloxane, die endständig ungesättigte organischen Reste tragen, eingesetzt werden, mit der Maßgabe, dass mindestens ein olefinischer Baustein enthalten ist, der mindestens zwei terminal ungesättigte Gruppen aufweist.

11. Verfahren nach Anspruch 10, **dadurch gekennzeichnet, dass** die Enzymimmobilisate **dadurch** mit einer Polyethersiliconbeschichtung versehen werden, dass die Enzymimmobilisate mit einer Reaktionsmischung, die Si-H-funktionelle Polysiloxane, mindestens eine terminal ungesättigte Gruppe tragende Polyether, gegebenenfalls endständige Kohlenstoff-KohlenstoffDoppelbindungen enthaltende Polysiloxane sowie einen die Hydrosilylierung katalysierenden Katalysator aufweist, unter Hydrosilylierungsbedingungen in Kontakt gebracht werden.

12. Verwendung von Enzympräparaten nach einem der Ansprüche 1 bis 9 als industrieller Biokatalysator.

## Claims

1. Enzyme preparations obtainable by providing enzyme immobilizates which comprise enzymes or microorganisms comprising enzymes immobilized on an inert support with a polyethersiloxane coating obtained by hydrosilylating Si-H-functional polysiloxanes, in which the olefinic units used are polyethers bearing at least one terminally unsaturated group and optionally additionally siloxanes which bear terminally unsaturated organic radicals, with the proviso that at least one olefinic unit which has at least two terminally unsaturated groups is present.

2. Enzyme preparations according to Claim 1, **characterized in that** the enzyme immobilizates comprise enzymes from the class of the hydrolases, preferably lipases.

3. Enzyme preparations according to either of Claims 1 and 2, **characterized in that** the inert supports have a particle size distribution in which 90% of the particles have a particle size of 10 to 5000 µm.

4. Enzyme preparations according to any one of Claims 1 to 3, **characterized in that** the inert supports used consist of silica, polyvinyl styrene, polymethacrylate or polyacrylate.

5. Enzyme preparations according to any one of Claims 1 to 4, **characterized in that** the Si-H-functional polysiloxanes used are Si-H polysiloxanes of the general formula (I) MₐD_{b}D'_{c}T_{d}Qₑ (I)
where
M = R¹₂R^{2a}SiO_{1/2}]
D = [R¹₂SiO_{2/2}]
D' = [R¹R^{2b}SiO_{2/2}]
Z' = [R¹SiO_{3/2}]
Q = [SiO_{4/2}]
N¹ =a + b + c + d + e = 3 to 850,
a = 2 to 10,
b = 1 to 800,
c = 0 to 400,
d = 0 to 10,
e = 0 to 10,
R¹ are the same or different and are each independently selected from the group comprising: saturated or unsaturated, optionally branched alkyl groups having 1 to 30 carbon atoms, alkaryl radicals having 7 to 30 carbon atoms, aryl radicals having 6 to 30 carbon atoms,
R^{2a} is independently hydrogen or R¹ and
R^{2b} is independently hydrogen or R¹.

6. Enzyme preparations according to any one of Claims 1 to 5, **characterized in that** the Si-H-functional polysiloxanes used are Si-H polysiloxanes of the general formula (I) where
N¹ - a + b + c + d + e = 6 to 160,
a= 2,
b= 2 to 150,
c= 2 to 75,
d= 0 and
e= 0.

7. Enzyme preparations according to any one of Claims 1 to 6, **characterized in that** the polyethers bearing at least one terminally unsaturated group used are those of the general formula (II): 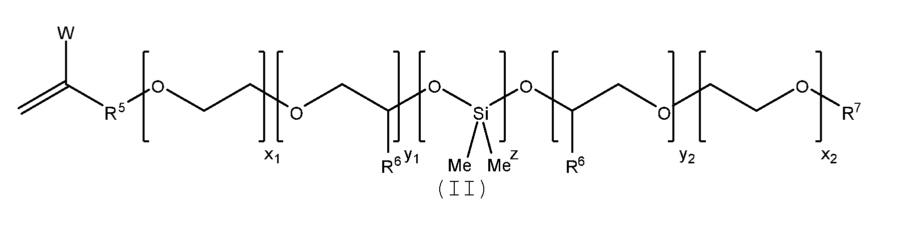 where
N² - x₁ + x₂ + y₁ + y₂ = 1 to 500,
x₁ = 0 to 200,
x₂ = 0 to 100,
y₁ = 0 to 200,
y₂ = 0 to 100,
z= 0 to 500,
W= hydrogen or methyl,
R⁵ is an optionally branched, optionally substituted alkylene radical which optionally bears double bonds and has 1 to 30 carbon atoms,
R⁶ is independently a radical selected from the group comprising methyl, ethyl and phenyl, and
R⁷ is an optionally branched, optionally substituted alkyl radical or carboxyl radical which optionally contains double bonds and has 1 to 30 carbon atoms, with the proviso that, when z = 0, x₂ = y₂ = 0.

8. Enzyme preparations according to any one of Claims 1 to 7, **characterized in that** the polysiloxanes containing terminal carbon-carbon double bonds used are polysiloxanes of the general formula (III) MₘDₙD'ₒTₚQ_{q} (III)
where
M= [R³₂R⁴SiO_{1/2}]
D= [R³₂SiO_{2/2}]
D' = [R³R⁴SiO_{2/2}]
T= [R³SiO_{3/2}]
Q= [SiO_{4/2}]
N² = m + n + o + p + q = 3 to 1000,
m= 2 to 10,
n = 1 to 800,
o= 0 to 20,
p = 0 to 10,
q= 0 to 10,
R³ are the same or different and are each independently selected from the group comprising: saturated or unsaturated, optionally branched alkyl groups having 1 to 30 carbon atoms, alkaryl radicals having 7 to 30 carbon atoms, aryl radicals having 6 to 30 carbon atoms, and
R⁴ is independently a terminally unsaturated alkyl radical, preferably vinyl, or an alkoxy radical or R₃.

9. Enzyme preparations according to any one of Claims 1 to 8, **characterized in that** the polysiloxanes containing terminal carbon-carbon double bonds used are those of the general formula (III) where
N² = m + n + o + p + q = 10 to 600,
m= 2,
n = 2 to 600,
o= 0,
p = 0,
q= 0 and
R⁴ is independently a terminally unsaturated alkyl radical.

10. Process for preparing enzyme preparations, **characterized in that** enzyme immobilizates which comprise enzymes or microorganisms comprising enzymes immobilized on an inert support are provided with a polyethersiloxane coating obtained by hydrosilylating Si-H-functional polysiloxanes, in which the olefinic units used are polyethers bearing at least one terminally unsaturated group and optionally additionally siloxanes which bear terminally unsaturated organic radicals, with the proviso that at least one olefinic unit which has at least two terminally unsaturated groups is present.

11. Process according to Claim 10, **characterized in that** the enzyme immobilizates are provided with a polyethersilicone coating by contacting the enzyme immobilizates, under hydrosilylation conditions, with a reaction mixture which comprises Si-H-functional polysiloxanes, polyethers bearing at least one terminally unsaturated group, optionally polysiloxanes containing terminal carbon-carbon double bonds, and also a catalyst which catalyses the hydrosilylation.

12. Use of enzyme preparations according to any one of Claims 1 to 9 as an industrial biocatalyst.

## Revendications

1. Préparations enzymatiques, pouvant être obtenues par le fait que des immobilisats d'enzymes, qui comprennent des enzymes, ou des micro-organismes contenant des enzymes, immobilisés sur un support inerte, sont pourvus d'un revêtement de polyéthersiloxane obtenu par hydrosilylation de polysiloxanes Si-H-fonctionnels, préparations dans lesquelles, en tant que constituants oléfiniques, on utilise au moins un polyéther portant un groupe à insaturation terminale, et éventuellement des siloxanes additionnels, qui portent des résidus organiques insaturés en position terminale, à la condition que soit présent au moins un constituant oléfinique qui comprend au moins deux groupes à insaturation terminale.

2. Préparations enzymatiques selon la revendication 1, **caractérisées en ce que** les immobilisats d'enzymes comprennent des enzymes de la classe des hydrolases, de préférence des lipases.

3. Préparations enzymatiques selon l'une des revendications 1 ou 2, **caractérisées en ce que** les supports inertes présentent une distribution granulométrique pour laquelle 90 % des particules ont une granulométrie de 10 à 5 000 µm.

4. Préparations enzymatiques selon l'une des revendications 1 à 3, **caractérisées en ce que** les supports inertes utilisés sont constitués de silice, de polyvinyl styrène, d e polyméthacrylate ou de polyacrylate.

5. Préparations enzymatiques selon l'une des revendications 1 à 4, **caractérisées en ce qu'**on utilise en tant que polysiloxanes Si-H-fonctionnels des Si-H-polysiloxanes de formule générale (I)
MₐD_{b}D'_{c}T_{d}Qₑ (I)
dans laquelle
M = [R¹₂R^{2a}SiO_{1/2}]
D = [R¹₂SiO_{2/2}]
D' = [R¹R^{2b}SiO_{2/2}]
T = [R¹SiO_{3/2}]
Q = [SiO_{4/2}]
N¹ = a + b + c + d + e = 3 à 850,
a = 2 à 10,
b = 1 à 800,
c = 0 à 400,
d = 0 à 10,
e = 0 à 10,
les radicaux R¹ sont, indépendamment les uns des autres, identiques ou différents et sont choisis dans le groupe comprenant les groupes alkyle ayant 1 à 30 atomes de carbone, saturés ou insaturés, éventuellement ramifiés, les radicaux alcaryle ayant 7 à 30 atomes de carbone, les radicaux aryle ayant 6 à 30 atomes de carbone,
les radicaux R^{2a} représentent chacun indépendamment des autres un atome d'hydrogène ou R¹, et
les radicaux R^{2b} représentent chacun indépendamment des autres un atome d'hydrogène ou R¹.

6. Préparations enzymatiques selon l'une des revendications 1 à 5, **caractérisées en ce qu'**on utilise en tant que polysiloxanes Si-H-fonctionnels des Si-H-polysiloxanes de formule générale (I), dans laquelle
N¹ - a + b + c + d + e = 6 à 160,
a = 2,
b = 2 à 150,
c = 2 à 75,
d = 0 et
e = 0.

7. Préparations enzymatiques selon l'une des revendications 1 à 6, **caractérisées en ce qu'**on utilise en tant qu'au moins un polyéther portant un groupe à insaturation terminale ceux de formule générale (II) 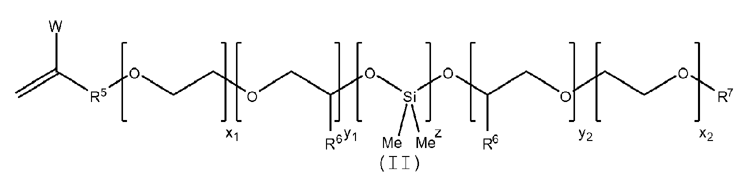 dans laquelle
N² - x₁ + x₂ + y₁ + y₂ = 1 à 500,
x₁ = 0 à 200,
x₂ = 0 à 100,
y₁ - 0 à 200,
y₂ = 0 à 100,
z = 0 à 500,
W est un atome d'hydrogène ou le groupe méthyle,
R⁵ est un radical alkylène ayant 1 à 30 atomes de carbone, éventuellement ramifié, éventuellement substitué, portant éventuellement des doubles liaisons, les radicaux R⁶ représentent chacun indépendamment des autres un radical choisi dans le groupe comprenant les groupes méthyle, éthyle ou phényle, et
R⁷ est un radical alkyle ou carboxyle ayant 1 à 30 atomes de carbone, éventuellement ramifié, éventuellement substitué, contenant éventuellement des doubles liaisons, à la condition que, quand z = 0, x₂ = y₂ = 0.

8. Préparations enzymatiques selon l'une des revendications 1 à 7, **caractérisées en ce qu'**on utilise en tant que polysiloxanes contenant des doubles liaisons carbone-carbone terminales des polysiloxanes de formule générale (III)
MₘDₙD'ₒTₚQ_{q} (III)
dans laquelle
M = [R³₂R⁴SiO_{1/2}]
D = [R³₂SiO_{2/2}]
D' = [R³R⁴SiO_{2/2}]
T = [R³SiO_{3/2}]
Q = [SiO_{4/2}]
N² - m + n + o + p + q = 3 à 1000,
m = 2 à 10,
n = 1 à 800,
o = 0 à 20,
p = 0 à 10,
q = 0 à 10,
les radicaux R³ sont indépendamment les uns des autres identiques ou différents et sont choisis dans le groupe comprenant les groupes alkyle ayant 1 à 30 atomes de carbone, saturés ou insaturés, éventuellement ramifiés, les radicaux alcaryle ayant 7 à 30 atomes de carbone, les radicaux aryle ayant 6 à 30 atomes de carbone, et les radicaux R⁴ représentent chacun indépendamment des autres un radical alkyle à insaturation terminale, de préférence le groupe vinyle, ou un radical alcoxyle, ou R₃.

9. Préparations enzymatiques selon l'une des revendications 1 à 8, **caractérisées en ce qu'**on utilise en tant que polysiloxanes contenant une double liaison carbone-carbone terminale ceux de formule générale (III) dans laquelle
N² - m + n + o + p + q = 10 à 600,
m = 2,
n = 2 à 600,
o = 0,
p = 0,
q = 0 et
les radicaux R⁴ représentent chacun indépendamment des autres un radical alkyle à insaturation terminale.

10. Procédé de fabrication de préparations enzymatiques, **caractérisé en ce qu'**on pourvoit des immobilisats d'enzymes, qui comprennent des enzymes ou des micro-organismes contenant des enzymes, immobilisés sur un support inerte, d'un revêtement de polyéthersiloxane obtenu par hydrosilylation de polysiloxanes Si-H-fonctionnels, dans lesquels, en tant que constituants oléfiniques, on utilise au moins un polyéther portant un groupe à insaturation terminale et éventuellement en outre des siloxanes qui portent des radicaux organiques insaturés en position terminale, à la condition qu'au moins un constituant oléfinique soit présent, qui comprend au moins deux groupes à insaturation terminale.

11. Procédé selon la revendication 10, **caractérisé en ce que** les immobilisats d'enzymes sont pourvus d'un revêtement de polyéthersilicone par le fait que les immobilisats d'enzymes sont, dans les conditions d'une hydrosilylation, mis en contact avec un mélange réactionnel qui comprend des polysiloxanes Si-H-fonctionnels, au moins un polyéther portant un groupe à insaturation terminale, éventuellement des polysiloxanes contenant des doubles liaisons carbone-carbone terminales, ainsi qu'un catalyseur catalysant l'hydrosilylation.

12. Utilisation des préparations enzymatiques selon l'une des revendications 1 à 9 en tant que biocatalyseur industriel.
